(19) Europäisches Patentamt / European Patent Office / Office européen des brevets

(11) **EP 3 479 801 A1**

(12) **EUROPEAN PATENT APPLICATION**
published in accordance with Art. 153(4) EPC

(43) Date of publication:
**08.05.2019 Bulletin 2019/19**

(51) Int Cl.:
*A61F 5/10* (2006.01)       *A61F 5/01* (2006.01)
*A61F 5/02* (2006.01)

(21) Application number: **17820318.8**

(22) Date of filing: **30.06.2017**

(86) International application number:
**PCT/JP2017/024092**

(87) International publication number:
**WO 2018/003964 (04.01.2018 Gazette 2018/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.07.2016 JP 2016132096**

(71) Applicants:
• **Furukawa Techno Material Co., Ltd.**
  **Kanagawa 254-0016 (JP)**
• **Furukawa Electric Co., Ltd.**
  **Chiyoda-ku**
  **Tokyo 100-8322 (JP)**
• **Tohoku University**
  **Sendai-shi, Miyagi 980-8577 (JP)**

(72) Inventors:
• **KISE, Sumio**
  **Hiratsuka-shi**
  **Kanagawa 254-0016 (JP)**

• **TANAKA, Toyonobu**
  **Hiratsuka-shi**
  **Kanagawa 254-0016 (JP)**
• **KAINUMA, Ryosuke**
  **Sendai-shi**
  **Miyagi 980-8577 (JP)**
• **OMORI, Toshihiro**
  **Sendai-shi**
  **Miyagi 980-8577 (JP)**
• **HATORI, Masahito**
  **Sendai-shi**
  **Miyagi 980-0803 (JP)**
• **KAMEDA, Tadakuni**
  **Sendai-shi**
  **Miyagi 980-0801 (JP)**
• **SUZUKI, Norifumi**
  **Sendai-shi**
  **Miyagi 980-0801 (JP)**

(74) Representative: **Forstmeyer, Dietmar et al**
  **BOETERS & LIECK**
  **Oberanger 32**
  **80331 München (DE)**

(54) **HALLUX VALGUS ORTHOSIS**

(57) {Technical problem} To provide a hallux valgus correction device that maximally reduces a feeling of strangeness (discomfort) to the patient at the time of wearing, suppresses pain by utilizing the characteristics of a superelastic alloy while a high corrective effect can be expected therefrom, and can be worn for a long time or for a long time period.

{Solution to problem} A hallux valgus correction device (1) for correcting hallux valgus, the hallux valgus correction device including: a corrector (10) made of a superelastic alloy; and a fixture (2, 3, and 4) formed from a fabric to attach the corrector, in which the corrector has a hinge part (11) that is rotationally movable in the bending direction and the stretching direction of one toe or a plurality of toes in need of correction.

*Fig. 1*

**Description**

TECHNICAL FIELD

The present invention relates to a hallux valgus orthosis, i.e. a bunion correction device.

BACKGROUND ART

Hallux valgus is a disease caused by anatomical factors (varus of hallux metatarsal bone, flat feet, and the like) and external factors (shoes, life style, and the like).

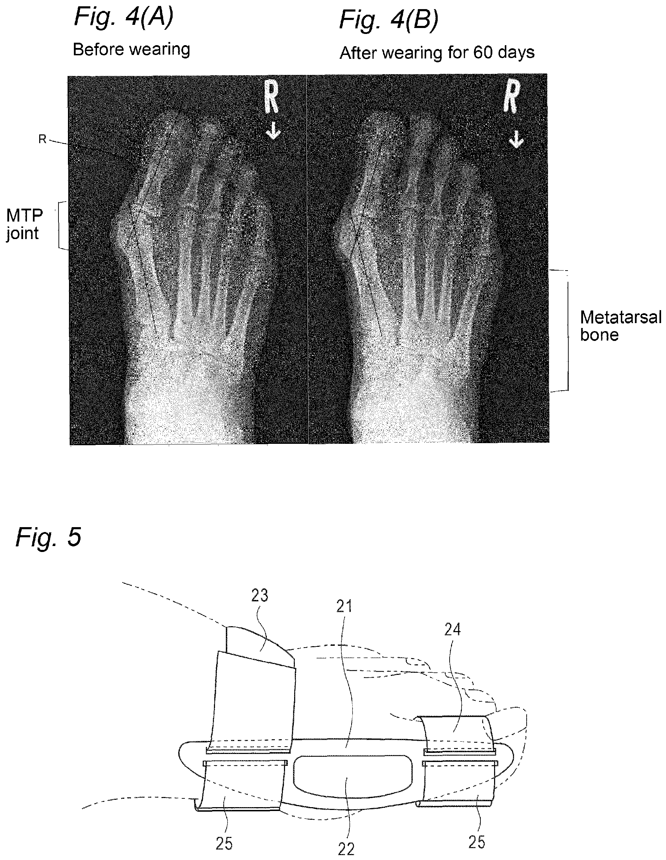

A case in which the angle of hallux valgus (R) shown in Fig. 4(A) and Fig. 4(B) is 9° or more and less than 15° is considered to be normal; a case in which the angle of hallux valgus is 15° or more and less than 20° is considered to be a mild case of a hallux valgus; a case in which the angle of hallux valgus is 20° or more and less than 40° is considered to be a moderate case of a hallux valgus; and a case in which the angle of hallux valgus is 40° or more is considered to be a severe case of a hallux valgus. There are patients who have mild cases of hallux valguses but suffer from great pain. Among patients with moderate cases of hallux valguses and severe cases of hallux valguses, when the pain cannot be controlled conservatively, many of the patients may need surgical operations. Under the current circumstances, a variety of devices for hallux valgus have been developed and sold in the market; however, many of them are devices for preservation, that is, devices for preventing aggravation of symptoms. Thus, no correction device with therapeutic effects exists.

Hallux valgus occurs mostly in women, and the patient gender ratio is believed to be one male to ten females. Furthermore, even if a patient recovers from the condition for the moment, when the patient restores the original lifestyle habit, the illness recurs.

Regarding currently available devices for correcting hallux valgus, there are arch or plantar supports (insoles) that are inserted in the sole at the time of walking in the daytime, and nighttime devices for correction that are worn at the time of sleeping. Examples of the nighttime devices include the following.

A schematic perspective view of the state of use of the nighttime device described in Patent Literature 1 is illustrated in Fig. 6. Fig. 6 is a perspective view for explaining the state of use of a conventional nighttime device for hallux valgus correction.

This nighttime device has a hinge (32) provided under the base of the big toe of a springy splint (31) extending across from the big toe to the inner side of the metatarsus. Furthermore, the nighttime device is a device that enables walking, in which the springy splint binds the big toe with a first annular fastening device (34) formed from a flexible and pliable material that does not easily stretch, for example, a fabric tape or a non-stretchable adhesive tape, and the springy splint binds the big toe and the metatarsus in the circumferential direction with a second annular fastening device (33) formed from a flexible and pliable material that does not easily stretch, for example, a fabric tape or a non-stretchable adhesive tape.

The shape of the springy splint (31) is a three-dimensional shape conforming to the shape of the foot. The material of the springy splint (31) is a metal or a plastic, and it is considered preferable to use a thin carbon fiber-reinforced sheet. A representative example is a springy splint made of a plastic.

A schematic perspective view of the state of use of the nighttime device described in Patent Literature 2 is illustrated in Fig. 5. Fig. 5 is a perspective view for explaining the state of use of another conventional nighttime device for hallux valgus correction.

This nighttime device is a device that uses a shape memory alloy for the splint (a correction sheet 21). Since the pliability of superelasticity is used, a corrective effect can be expected as long as the device can be worn for a long time. In the diagram, reference numeral 22 represents a space provided in a correction sheet (21); reference numeral 23 represents an annular fastening device for the metatarsal bone; reference numeral 24 represents an annular fastening device for the big toe; and reference numerals 25 and 25 are hook-and-loop fasteners.

CITATION LIST

PATENT LITERATURES

Patent Literature 1: Japanese Patent No. 4917752
Patent Literature 2: Japanese Patent No. 5369321

SUMMARY OF INVENTION

TECHNICAL PROBLEM

Since the nighttime device of Patent Literature 1 provides a dramatic improvement in the walking performance, the device is made wearable also in times other than the nighttime. However, while it is said that the springy splint has springiness, since the springy splint (31) has a three-dimensionally shaped hinge (32), the periphery of the hinge area is rigid. Since the springy splint has a structure in which as a portion is farther away from the hinge part, the portion gradually becomes to have spring property, no load can be applied to the entire inner foot side. As such, when only the vicinity of the annular fastening device (34) is bent, a satisfactory corrective effect cannot be obtained.

Furthermore, since the materials for the annular fastening devices (33 and 34) are configured to include a fabric tape or a non-stretchable adhesive tape, when the annular fastening devices come into direct contact with the skin, the patient may have a feeling of strangeness (discomfort). Therefore, it is difficult to wear the device for a long time (for example, overnight wearing).

The nighttime device of Patent Literature 2 applies a correction sheet (21) along the direction of bending or stretching of the big toe, and therefore, movement of the big toe is restricted. Thus, wearing of the device for a long time is difficult.

Furthermore, since the materials for the annular fastening devices (23 and 24) are representatively configured to include a supporter, when the annular fastening devices come into direct contact with the skin, the patient may have a feeling of strangeness (discomfort). Therefore, it is difficult to wear the device for a long time (for example, overnight wearing).

As such, regarding the conventional hallux valgus correction devices intended mainly for nighttime wearing, it has been difficult for users to wear the devices for a long time, because the users feel painful or have an unpleasant feeling of wearing. Furthermore, for example, the joining of a metal (shape memory alloy) part and a supporter may be dislocated because the joining force is weak, or the fixing of the big toe may be dislocated because the fixing force is weak. Even in the case of walking to the bathroom at night, the device may be dislocated, and in some severe cases, the device is broken. Furthermore, since the supporter gets sweaty, the supporter gives a feeling of strangeness to the skin, and the duration of wearing is limited to 2 to 3 hours. Thus, wearing the device overnight has been torments for patients. For example, one of the causative factors for sleep disorders may be restless legs syndrome. The restless legs syndrome is caused by discomfort or pain in the legs, and discomfort or pain in the legs has been mentioned as a causative factor for sleep disorders. The discomfort caused by wearing of a hallux valgus correction device is quite different from the cause of onset. However, since discomfort or pain in the legs significantly affects sleep, it is important for a nighttime device to reduce the pain and a feeling of strangeness at the time of wearing.

Thus, the present invention is contemplated for providing a hallux valgus correction device that maximally reduces a feeling of strangeness (discomfort) to the patient at the time of wearing, suppresses pain by utilizing the characteristics of a superelastic alloy while a high corrective effect can be expected therefrom, and can be worn for a long time or for a long time period.

SOLUTION TO PROBLEM

The inventors of the present invention have conducted a thorough investigation in order to solve the problems described above, and as a result, the inventors have found that a hallux valgus correction device that maximally reduces a feeling of strangeness (discomfort) at the time of wearing, suppresses pain by utilizing the characteristics of a superelastic alloy while a high corrective effect can be expected, and can be worn for a long time or for a long time period, is obtained by providing a hinge part at the base of the big toe of a sheet made of a superelastic alloy and making the hinge part movable.

That is, the present invention is to provide the following means:

(1) A hallux valgus correction device for correcting hallux valgus,
the hallux valgus correction device including:

a corrector made of a superelastic alloy; and
a fixture formed from a fabric to attach the corrector,
in which the corrector has a hinge part that is rotationally movable in the bending direction and the stretching direction of one toe or a plurality of toes in need of correction.

(2) The hallux valgus correction device according to item (1), in which the corrector made of the superelastic alloy is formed from a Cu-Al-Mn-based superelastic alloy.

(3) The hallux valgus correction device according to item (1) or (2), in which the fixture has a first annular bandage enclosing the big toe; a second annular bandage enclosing the metatarsus; and a corrector storage unit that extends over the first annular bandage and the second annular bandage and accommodates the corrector in the inside.

(4) The hallux valgus correction device according to any one of items (1) to (3), wherein in the fabric, a portion that comes into direct contact with the skin is formed from an extra-fine denier resin fiber.

(5) The hallux valgus correction device according to any one of items (1) to (4), in which the fixture includes a cushioning member between the hinge part and the big toe joint.

(6) The hallux valgus correction device according to any one of items (1) to (5), in which the corrector is comprised of a Cu-Al-Mn-based superelastic alloy having a composition containing 3.0 to 10.0 mass% of Al, and 5.0 to 20.0 mass% of Mn, with the balance being Cu and unavoidable impurities, and capable of containing, as an optional additional element, 0.000 to 10.000 mass% in total of at least one selected from the group consisting of Ni, Co, Fe, Ti, V, Cr, Si, Nb, Mo, W, Sn, Mg, P, Be, Sb, Cd, As, Zr, Zn, B, C, Ag, and misch metal (Pr, Nd, and the like).

EFFECTS OF INVENTION

According to the hallux valgus correction device of the present invention, by providing a hinge part at the base of the big toe of a superelastic alloy sheet and making the hinge part movable, the hallux valgus correction device can be provided, which maximally reduces a feeling of strangeness (discomfort) of the patient at the time of wearing, which suppresses pain while a high corrective effect can be expected, and which can be worn for a long time or for a long time period.

Other and further features and advantages of the invention will appear more fully from the following description, appropriately referring to the accompanying drawings.

BRIEF DESCRIPTION OF THE DRAWINGS

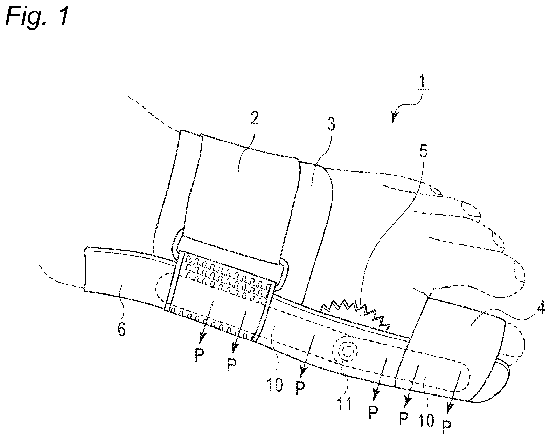

{Fig. 1}
Fig. 1 is a perspective view explaining the state of use of the hallux valgus correction device of an embodiment according to the present invention.
{Fig. 2}
Fig. 2 is a front view explaining the state of use of the hallux valgus correction device of the above-described embodiment according to the present invention.
{Fig. 3}
Fig. 3 is a front view explaining the developed state of the hallux valgus correction device of the above-described embodiment according to the present invention.
{Fig. 4}
Fig. 4(A) and Fig. 4(B) are photographs showing the roentgenologically measured angle (angle of hallux valgus, R) of hallux valgus, and Fig. 4(A) shows the condition before wearing of the hallux valgus correction device, while Fig. 4(B) shows the condition after treating hallux valgus by wearing the hallux valgus correction device.
{Fig. 5}
Fig. 5 is a perspective view explaining the state of use of the hallux valgus correction device of a conventional example.
{Fig. 6}
Fig. 6 is a perspective view explaining the state of use of the hallux valgus correction device of another conventional example.
{Fig. 7}
Fig. 7 is a schematic diagram explaining an example of the correlation between the amount of bending deformation and the bending load of the superelastic alloy according to the present invention.
{Fig. 8}
Fig. 8 is a schematic diagram explaining an example of a production process of the superelastic alloy according to the present invention.

MODE FOR CARRYING OUT THE INVENTION

The hallux valgus correction device of the present invention will be explained using the drawings. As illustrated in Fig. 1, the hallux valgus correction device (1) of an embodiment according to the present invention includes: a corrector (10) formed from a superelastic alloy; a fixture (3) that fastens the corrector to the metatarsus; and a fixture (4) that fastens the corrector to the big toe. Furthermore, the hallux valgus correction device of an embodiment according to the present invention includes: a fastening band (2) that fixes the fixture (3) and the corrector (10) to the foot, on the outer side of the fixture (3). The hallux valgus correction device of an embodiment according to the present invention includes: a big-toe-base inner-side protrusion cushioning pad (a cushioning member) (5) at the part that contacts the base (joint) of the big toe.

The corrector (10) is accommodated (stored) in a corrector storage bag (6). Furthermore, the corrector (10) has a hinge part (11) that can rotationally move in the bending direction and stretching direction of the toe that is in need of correction (that is, big toe). It is enough that the hinge part (11) is provided at least in an area in the vicinity of the big toe base, and the hinge part may be further provided in another joint area of the big toe.

By adopting such a configuration, the hallux valgus correction device of an embodiment according to the present invention can exert force (P) on the entirety of the corrector (from the big toe to the metatarsus), with the big toe joint serving as a fulcrum. As a result, there is no force that is partially exerted on the big toe, and therefore, the correction device can be worn for a long time or a for a long time period without causing any feeling of strangeness (discomfort), and the correction device can correct hallux valgus effectively.

When using an integrated material (for example, one sheet of the sheet) of a superelastic alloy that does not have any hinge, a certain force can be applied to the entire inner side of the foot by superelasticity, and correction can be achieved without causing a pain. However, since the toe of the foot cannot be bent, walking is made impossible, and also, the discomfort caused by unintentional moving of toes to cause dislocation of the device in sleeping as well as dislocation of the device is much more severe than one can imagine.

In this regard, according to the present invention, when the superelastic alloy sheet (the corrector 10) and a hinge (11) are combined, to make the hinge (11) movable and rotationally movable, two superelastic alloy sheets (10 and 10) can conform to the angle of bending of the toe by means of the hinge (11). Thereby, the burden on the patient is reduced, and the correction device can be worn for a long time or for a long time period. Thus, the corrective effect of superelasticity can be exhibited to a high level.

In regard to the hallux valgus correction device of the present invention, although not illustrated in the diagram, it is desirable that the hinge (11) joins two superelastic alloy sheets (10 and 10) in a state that the two superelastic alloy sheets do not move vertically or horizontally, but each of them can freely move rotationally. Meanwhile, one of the two superelastic alloy sheets (10 and 10) has a length approximately equal to that of the big toe, and the other one has a length approximately equal to that of the long bone.

In other words, according to the present invention, even though the corrector (10) made of a superelastic alloy has a hinge (11), since the entirety of the corrector has a predetermined elastic force, the corrector can exert a corrective force (P) on the big toe. This is also the same even if the hinge part is in a bent state. It is the technical significance that a certain corrective force (P) can be exerted on the range from the big toe to the metatarsus without causing a feeling of strangeness (discomfort) (even both at the time of walking and in the nighttime).

Here, "sheets made of a superelastic alloy" must be used because an elastic force should be exhibited (force P described in the following paragraph 0017) and deformation should be prevented.

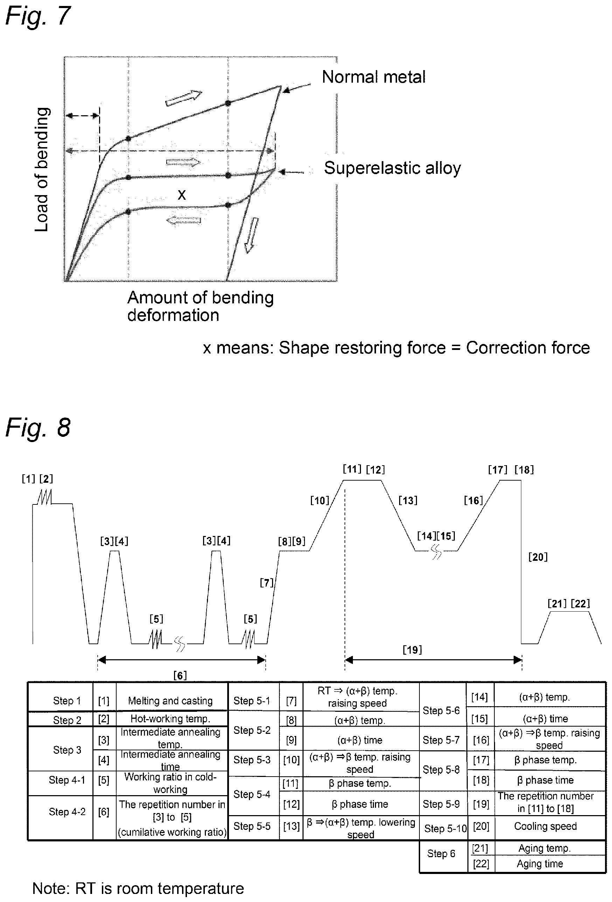

As illustrated in Fig. 7, in the case of a common metal, restoration of the shape occurs as long as the metal is in the elastic deformation region. However, when a load is applied to reach the plastic deformation region, the metal remains deformed and cannot restore the original shape. When the corrector is subjected to an abrupt force due to walking or the like and is deformed, the corrector loses its corrective force, and in the worst case, the user may be hurt.

In regard to hallux valgus correction devices, disadvantages of existing products can be roughly divided into two (the pain in the big toe is also a kind of feeling of strangeness; however, this will be also divided into two).

- Pain: Hallux valgus itself is painful.
- Feeling of strangeness: Discomfort caused by the structure/configuration of the device.

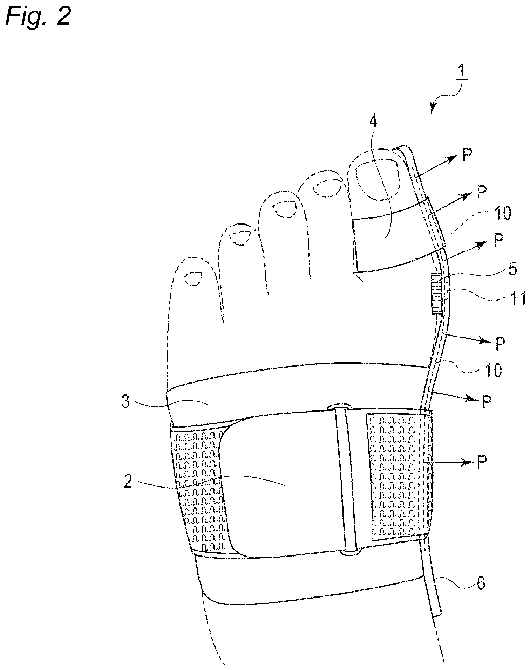

A superelastic alloy is such that when the amount of deformation is beyond a certain level, the load becomes constant, and a superelastic alloy has a lower rigidity, with the Young's modulus being 1/3 of that of iron. Therefore, the superelastic sheets (corrector 10) do not forcibly bend the big toe but fit themselves so as to stick to the big toe as a whole, and a constant moderate force (P) is applied to the big toe as a whole. Therefore, the superelastic sheets allow the user to feel pain to a significantly reduced degree (see Fig. 1 and Fig. 2).

On the other hand, at bedtime, toes are unintentionally bent, or toes are bent upon walking to go to the bathroom. However, if the toes cannot be bent, this causes the feeling of strangeness (discomfort) that is much severe than one can imagine.

The hallux valgus correction device of the present invention makes the MTP joint (see Fig. 4(A) and Fig. 4(B)) smooth, with a sensation that allows the user to forget the fact that he/she is wearing the device, and also improves the angle (R) of hallux valgus. Thus, the correction device forms 'the arch of the foot' ('the arch of the foot' has the shape of a transverse cross-section of the foot, and a state in which two ends of the big toe (first toe) and the little toe (fifth toe) reliably grasp the ground surface, and the transverse cross-section of the foot forms an arch, is regarded ideal), and thereby walking becomes comfortable.

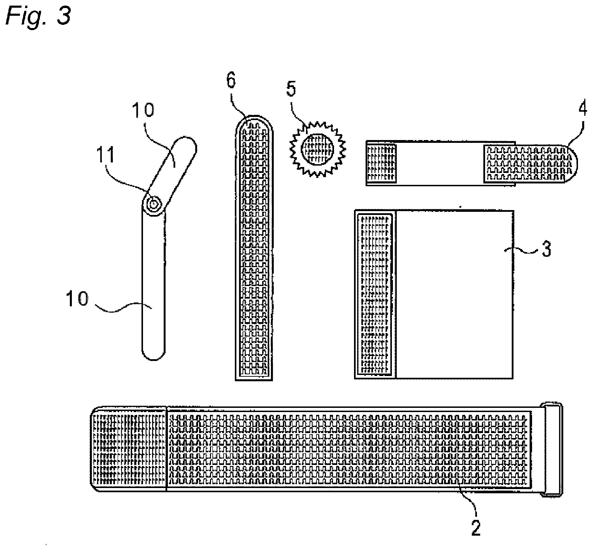

Regarding the hallux valgus correction device of the present invention, monitors gave the following mentions on the use of a corrector (10) made of a superelastic alloy and having a hinge part (11) as illustrated in Fig. 1, Fig. 2, and Fig. 3.

(1) The hallux valgus correction device has corrective force, while the feeling of wearing is natural.
(2) The band on the dorsum of the foot has a good feeling of fixation or insertion.
(3) Since the corrector made of a superelastic alloy is movable, walking while wearing the device is made easy.
(4) Compared to conventional devices, there is no feeling of strangeness at the time of wearing.

Furthermore, orthopedists gave the following mentions (opinions).

(5) Since the user can have a sound sleep, the correction device is an unprecedented, true nighttime device that can be worn in the nighttime.
(6) Walking is enabled, and thus, the device can be worn in the daytime, too.
(7) The correction device can be worn in the nighttime and in the daytime, the device can be worn for a long time, and a therapeutic effect can be expected.

The hinge (11) can be installed such that one corrector (10) can move around 360° (that is, rotate) with respect to the other corrector (10). At the time of wearing, it is desirable that the hinge actually moves around up to about 90°, that is, up to the angle at which the toe can be bent.

Here, the hinge (11) means an object in which two superelastic alloy sheets (10 and 10) are riveted at the center of rotation, as illustrated in the drawings.

According to the hallux valgus correction device of the present invention, the following can be expected in connection with the therapeutic effect described above. That is, pain can be reduced by inducing contracture of the MTP joint (see Fig. 4(A) and Fig. 4(B)). This is speculated, because alignment of the big toe and the proximal phalanx is normalized by obtaining a corrective effect, and consequently, normalization of the load balance on the foot is achieved.

Furthermore, in the hallux valgus correction device of the present invention, since a fabric of an extra-fine denier resin fiber as illustrated in Fig. 1, Fig. 2, and Fig. 3, for example, an extra-fine denier polyester fiber (manufactured by TEIJIN LIMITED, trade name: NANOFRONT), is used in a part that comes into direct contact with the skin, the following advantages are obtained.

(1) Since the amount of moisture evaporation is small and the stratum corneum is not damaged, the fabric feels good to the touch, and there is no feeling of strangeness (discomfort).
(2) Since the surface area is made large by the extra-fine denier fibers, the fabric is water-absorbent. The fabric absorbs sweat and does not get sweaty.
(3) Even if the user does exercise, the temperature is not likely to rise. Therefore, even if the correction device is worn in sleep, the fabric does not get sweaty.
(4) Since the fabric has high friction resistance, the correction device does not easily slip away and is not likely to be dislocated.

Meanwhile, the fabric to be used for the hallux valgus correction device of the present invention is not limited to the above-described extra-fine denier polyester fiber. Any fabric having an equivalent effect can be used without being limited to the extra-fine denier polyester fiber.

In regard to the hallux valgus correction device of the present invention, it is preferable that the portion that comes into direct contact with the skin in the fabric is formed from an extra-fine denier polyester fiber. Here, examples of the portion that comes into direct contact with the skin include: the inner side portion of the fixture (3) (annular bandage for the metatarsal bone); and the inner side portion of the fixture (4) (annular bandage for the big toe).

Furthermore, there are no particular limitations on the materials for the fixture (3) (metatarsal bone fastening belt), the big-toe-base inner-side protrusion cushioning pad (5), and the corrector storage bag (6). In the embodiment that will be described below, these are made of synthetic rubber (for example, made of NEOPRENE). However, it is also acceptable that the entirety of the fixture including these is formed from an extra-fine denier polyester fiber.

NANOFRONT is the trade name for an extra-fine denier polyester fiber manufactured by TEIJIN LIMITED, with one strand of the fiber having a diameter of about 700 nm.

Preferably, the hallux valgus correction device of the present invention has: a fastening band (2) that is retained by a first annular bandage (4) at the big toe part and a second annular bandage (3) at the metatarsal part, and fastens the metatarsal part to the outer side of the second annular bandage at the metatarsal part. It is also preferable that the hallux valgus correction device of the present invention has a cushioning pad (cushioning member) (5) on the big toe joint inner side, and the base of the foot and the big toe are retained by these fixtures (first and second annular bandages) (3 and 4). It is also preferable that the hallux valgus correction device has a corrector (10) of sheets made of superelastic alloy, which is accommodated inside the corrector storage bag (6) and extends along the inner side of the foot.

By adopting such a configuration, the contact area between the hallux valgus correction device and the foot can be reduced, and the feeling of strangeness of the device can be reduced.

Furthermore, by providing a cushioning member (5) between the hinge part (11) and the big toe joint, the feeling of strangeness of the hallux valgus correction device can be further reduced. Furthermore, by providing a cushioning member (5) and thereby providing a space between the big toe joint and the corrector (10), the elastic force (P) of the corrector (10) can be exerted more efficiently on the big toe.

The hallux valgus correction device of the present invention illustrated in Fig. 1, Fig. 2, and Fig. 3 is configured to include these first annular bandage (4), second annular bandage (3), fastening band (2), cushioning pad (5), and corrector storage bag (6), as separate bodies.

Preferably, the hallux valgus correction device of the present invention may be configured to include a fixture having the first annular bandage (4), the second annular bandage (3), the fastening band (2), and the cushioning pads (5) sewed in, for example, the corrector storage bag (6) as an integrated body.

In regard to the hallux valgus correction device of the present invention, the kind of the superelastic alloy that constitutes the corrector (10) and, if necessary, the hinge part (11), is not particularly limited. For example, use can be made of any of various superelastic alloys, such as a Ni-Ti-based alloy and a Cu-Al-Mn-based alloy. Among these, it is preferable to use a Cu-Al-Mn-based alloy as the superelastic alloy. A preferred composition thereof and the like will be described below.

<Composition of Cu-Al-Mn-based alloy>

The copper-based alloy to be used in the present invention having shape memory characteristics and superelasticity is an alloy containing Al and Mn. This alloy becomes a $\beta$ phase (body-centered cubic) single phase (in the present specification, which may be simply referred to as $\beta$ single phase) at high temperature, and becomes a two-phase microstructures of a $\beta$ phase and an $\alpha$ phase (face-centered cubic) (in the present specification, which may be simply referred to as ($\alpha+\beta$) phase) at low temperature. The temperatures ranges may vary depending on the alloy composition, but the high temperature at which the $\beta$ single phase is obtained is usually 700°C or higher, and the low temperature at which the ($\alpha+\beta$) phase is obtained is usually less than 700°C.

The Cu-Al-Mn-based alloy to be used in the present invention has a composition containing 3.0 to 10.0 mass% of Al and 5.0 to 20.0 mass% of Mn, with the balance being Cu and unavoidable impurities. If the content of elemental Al is too small, the $\beta$ single phase cannot be formed, and if the content is too large, the alloy becomes brittle. The content of elemental Al may vary depending onto the content of elemental Mn, but a preferred content of elemental Al is 6.0 to 10.0 mass%. When the alloy contains elemental Mn, the range of existence of the $\beta$ phase extends to a lower Al-content side, and cold-workability is markedly enhanced. Thus, forming work is made easier. If the amount of addition of elemental Mn is too small, satisfactory workability is not obtained, and the region of the $\beta$ single phase cannot be formed. Also, if the amount of addition of elemental Mn is too large, sufficient shape recovery characteristics are not obtained. A preferred content of Mn is 8.0 to 12.0 mass%.

In addition to the essential alloying elements described above, the Cu-Al-Mn-based alloy to be used in the present invention can further contain, optional additionally alloying element(s), at least one selected from the group consisting of Ni, Co, Fe, Ti, V, Cr, Si, Nb, Mo, W, Sn, Mg, P, Be, Sb, Cd, As, Zr, Zn, B, C, Ag and misch metal (for example, Pr and Nd). These elements exhibit an effect of enhancing the physical strength of the Cu-Al-Mn-based alloy, while maintaining cold-workability. The content in total of any of these optional additionally elements is preferably 0.001 to 10.000 mass%, and particularly preferably 0.001 to 5.000 mass%. If the content of any of these optional additionally elements is too large, the martensite transformation temperature is lowered, and the $\beta$ single phase microstructure becomes unstable.

Ni, Co, Fe and Sn are elements that are effective for strengthening of the matrix microstructure. Co makes the grains coarse by forming Co-Al intermetallic compound, but Co in an excess amount causes lowering of toughness of the resultant alloy. A content of Co is 0.001 to 2.000 mass%. A content of Ni and Fe is respectively 0.001 to 3.000 mass%. A content of Sn is 0.001 to 1.000 mass%.

Ti is bonded to N and O, which are inhibitory elements, to form oxynitride. Also, Ti forms boride when added in combination with B, to enhance physical strength. A content of Ti is 0.001 to 2.000 mass%.

V, Nb, Mo and Zr have an effect of enhancing hardness, to enhance abrasion resistance. Further, since any of these elements are hardly solid-solubilized into the matrix, the elements precipitate as a $\beta$ phase (bcc crystals), to enhance physical strength. Contents of V, Nb, Mo and Zr are respectively 0.001 to 1.000 mass%.

Cr is an element effective for retaining abrasion resistance and corrosion resistance. A content of Cr is 0.001 to 2.000 mass%. Si has an effect of enhancing corrosion resistance. A content of Si is 0.001 to 2.000 mass%. W is hardly solid-solubilized into the matrix, and thus has an effect of precipitation strengthening. A content of W is 0.001 to 1.000 mass%.

Mg has an effect of eliminating N and O, which are inhibitory elements, fixes S that is an inhibitory element as sulfide, and has an effect of enhancing hot-workability or toughness. Addition of a large amount of Mg brings about grain boundary segregation, and causes embrittlement. A content of Mg is 0.001 to 0.500 mass%.

P acts as a de-acidifying agent, and has an effect of enhancing toughness. A content of P is 0.01 to 0.50 mass%. Be, Sb, Cd, and As have an effect of strengthening the matrix microstructure. Contents of Be, Sb, Cd, and As are respectively 0.001 to 1.000 mass%.

Zn has an effect of raising the shape memory treatment temperature. A content of Zn is 0.001 to 5.000 mass%. When appropriate amounts of B and C are used, a pinning effect is obtained, and thereby an effect of coarsening the grains is obtained. Particularly, combined addition of B and C together with Ti and Zr is preferred. Contents of B and C are respectively 0.001 to 0.500 mass%.

Ag has an effect of enhancing cold-workability. A content of Ag is 0.001 to 2.000 mass%. When an appropriate amount of misch metal is used, a pinning effect is obtained, and thereby an effect of coarsening the grains is obtained. A content of misch metal is 0.001 to 5.000 mass%. Misch metal refers to an alloy of rare earth elements, such as La, Ce, and Nd, for which separation into simple substances is difficult.

<Method of producing Cu-Al-Mn-based alloy>

In regard to the Cu-Al-Mn-based alloy to be used in the present invention, regarding the production conditions for obtaining a superelastic alloy which stably provides satisfactory superelastic characteristics and has excellent resistance to repeated deformations, a production process such as described below may be mentioned.

The production process of the Cu-Al-Mn-based alloy is mainly composed of, as illustrated in Fig. 8, melting and casting [Step 1], hot-forging and hot-working [Step 2], intermediate annealing [Step 3], cold-working [Step 4], shape memory heat treatment [Step 5], and aging treatment [Step 6].

In the entire production process, particularly, when the heat treatment temperature [3] for intermediate annealing [Step 3] is set to the range of 400°C to 680°C, and the cold-working ratio or the working ratio for cold wire-drawing [5] for the cold-work (specifically, cold-rolling or cold-wire-drawing) [Step 4-1] is set to the range of 30% or more, a Cu-Al-Mn-based alloy which stably provide satisfactory superelastic characteristics is obtained. In addition to those, in the shape memory heat treatment [Step 5-1] to [Step 5-10], the speeds of temperature raising [10] and [16] in heatings [Step 5-3] and [Step 5-7] from the temperature ranges [8] and [14] for obtaining the $(\alpha+\beta)$ phase (which may vary depending on the alloy composition, but generally 400°C to 650°C, and preferably 450°C to 550°C) to the temperature ranges [11] and [17] for obtaining the $\beta$ single phase (which may vary depending on the alloy composition, but generally 700°C or higher, preferably 750°C or higher, and more preferably 900°C to 950°C), are each controlled to a predetermined slow range, such as 0.1°C/min to 20°C/min. In addition, the speed of temperature lowering [13] in cooling [Step 5-5] from the temperature range [11] for obtaining the $\beta$ single phase to the temperature range [14] for obtaining the $(\alpha+\beta)$ phase, is controlled to a predetermined slow range, such as 0.1°C/min to 20°C/min. Further, after the heating [Step 5-3] from the temperature range [8] for obtaining the $(\alpha+\beta)$ phase to the temperature range [11] for obtaining the $\beta$ single phase, a series of steps including: from retention [Step 5-4] in a temperature range [11] for obtaining the $\beta$ single phase for a predetermined time [12]; cooling [Step 5-5] from the temperature range [11] for obtaining the $\beta$ single phase to the temperature range [14] for obtaining the $(\alpha+\beta)$ phase at a speed of temperature lowering [13] of 0.1°C/min to 20°C/min; retention [Step 5-6] in the temperature range [14] for a predetermined time [15]; heating [Step 5-7] from the temperature range [14] for obtaining the $(\alpha+\beta)$ phase to the temperature range [17] for obtaining the $\beta$ single phase at a speed of temperature raising [16] of 0.1 °C/min to 20°C/min; to retention [Step 5-8] in the temperature range [17] for a predetermined time [18], that is, a series including from [Step 5-4] to [Step 5-8], is repeated at least one time and preferably at least four times (Step [5-9]). Thereafter, rapid cooling [Step 5-10] is carried out lastly.

Preferably, a production process such as follows may be mentioned.

In a usual manner, after melting and casting [Step 1] and hot-working [Step 2] of hot-rolling or hot-forging is carried out, intermediate annealing [Step 3] at 400°C to 680°C [3] for 1 to 120 minutes [4], and then cold-working [Step 4-1] of cold-rolling or cold-wire-drawing at a working ratio of 30% or higher [5] are carried out. Herein, the intermediate annealing [Step 3] and the cold-working [Step 4-1] may be carried out once each in this order, or may be repeated [Step 4-2] in this order at a number of repetitions [6] of two or more times. Thereafter, the shape memory heat treatment [Step 5-1] to [Step 5-10] is carried out.

The shape memory heat treatment [Step 5-1] to [Step 5-10] includes: heating [Step 5-3] from a temperature range [8] for obtaining an $(\alpha+\beta)$ phase (for example, 450°C) to a temperature range [11] for obtaining a $\beta$ single phase (for example, 900°C) at a speed of temperature raising [10] of 0.1°C/min to 20°C/min, preferably 0.1°C/min to 10°C/min, and more preferably 0.1°C/min to 3.3°C/min (hereinafter, referred to a slow temperature raising); retention [Step 5-4] at that heating temperature [11] for 5 minutes to 480 minutes, and preferably 10 to 360 minutes [12]; cooling [Step 5-5] from a temperature range [11] for obtaining a $\beta$ single phase (for example, 900°C) to a temperature range [14] for obtaining an $(\alpha+\beta)$ phase (for example, 450°C) [14] at a speed of temperature lowering [13] of 0.1 °C/min to 20°C/min, preferably 0.1 °C/min to 10°C/min, and more preferably 0.1°C/min to 3.3°C/min (hereinafter, referred to a slow temperature lowering); and retention [Step 5-6] at that temperature [14] for 20 to 480 minutes, and preferably 30 to 360 minutes [15]. Thereafter, the resultant alloy is subjected to: the heating [Step 5-7] again from a temperature range [14] for obtaining an ($\alpha$+$\beta$) phase (for example, 450°C) to a temperature range [17] for obtaining a $\beta$ single phase (for example, 900°C) at the speed of temperature raising [16] of the slow temperature raising; and retention [Step 5-8] at that temperature [17] for 5 to 480 minutes, and preferably 10 to 360 minutes [18]. Repetition [Step 5-9] of such slow temperature lowering [13] [Step 5-5] and slow temperature raising [16] [Step 5-7] is carried out at the number of the repetitions [19] of at least one time and preferably at least four times. Then, the shape memory heat treatment includes: rapid cooling [Step 5-10], for example, water cooling.

The temperature range for obtaining an ($\alpha$+$\beta$) single phase and the temperature range to be determined in the present invention is set to 400°C to 650°C, and preferably 450°C to 550°C.

The temperature range for obtaining a $\beta$ single phase is set to 700°C or higher, preferably 750°C or higher, and more preferably 900°C to 950°C.

After the shape memory heat treatment [Step 5-1] to [Step 5-10], it is preferable to perform aging heat treatment [Step 6] at 100°C to 200°C [21] for 5 to 120 minutes [22]. If the aging temperature [21] is too low, the $\beta$ phase is unstable, and if the resultant alloy is left to stand at room temperature, the martensite transformation temperature may change. On the contrary, if the aging temperature [21] is slightly higher, bainite (a microstructure) or it is too high, precipitation of the $\alpha$ phase occurs. In particular, the shape memory characteristics or superelasticity tends to be worsened conspicuously, due to the occurrence of precipitation of $\alpha$ phase.

By repeatedly performing [Step 4-2] intermediate annealing [Step 3] and cold-working [Step 4-1], the crystalline orientation can be integrated more preferably. The number of repetitions [6] of intermediate annealing [Step 3] and cold-working [Step 4-1] may be one time, but is preferably two or more times, and more preferably three or more times. This is because, as the number of repetitions [6] of the intermediate annealing [Step 3] and the cold-working [Step 4-1] is larger, the characteristics can be enhanced.

(Preferred conditions for the steps)

The intermediate annealing [Step 3] is carried out at 400°C 680°C [3] for 1 minute to 120 minutes [4]. It is preferable that this intermediate annealing temperature [3] is set to a lower temperature, and preferably to 400°C to 550°C.

The cold-working [Step 4-1] is carried out at a working ratio [5] of 30% or higher. Herein, the working ratio is a value defined by formula:

$$\text{Working ratio (\%)} = \{(A_1-A_2)/A_1\} \times 100$$

wherein $A_1$ represents the cross-sectional area of a specimen obtained before cold-working (cold-rolling or cold-wire-drawing); and $A_2$ represents the cross-sectional area of the specimen obtained after cold-working.

The cumulative working ratio ([6]) in the case of repeatedly performing this intermediate annealing [Step 3] and cold-working [Step 4-1] two or more times is preferably set to 30% or higher, and more preferably 45% or higher. There are no particular limitations on the upper limit of the cumulative working ratio, but the cumulative working ratio is usually 95% or lower.

In regard to the shape memory heat treatment [Step 5-1] to [Step 5-10], first, in [Step 5-1], temperature raising is carried out after the cold-working, from room temperature to a temperature range [8] for obtaining an ($\alpha$+$\beta$) phase (for example, 450°C) at the speed of temperature raising [7] (for example, 30°C/min). Then, retention [Step 5-2] is performed in the temperature range [8] for obtaining the ($\alpha$+$\beta$) phase (for example, 450°C) for 2 to 120 minutes, and preferably 10 to 120 minutes [9]. Then, when heating [Step 5-3] is performed from the temperature range [8] for obtaining the ($\alpha$+$\beta$) phase (for example, 450°C) to the temperature range [11] for obtaining the $\beta$ single phase (for example, 900°C), the speed of temperature raising [10] is set to 0.1 °C/min to 20°C/min, preferably 0.1 °C/min to 10°C/min, and more preferably 0.1 °C/min to 3.3°C/min, of the slow temperature raising. Then, the alloy is retained [Step 5-4] in this temperature range [11] for 5 to 480 minutes, and preferably 10 to 360 minutes [12]. Then, cooling [Step 5-5] is performed from the temperature range [11] for obtaining the $\beta$ single phase (for example, 900°C) to the temperature range [14] for obtaining the ($\alpha$+$\beta$) phase (for example, 450°C) at the speed of temperature lowering [13] of 0.1 °C/min to 20°C/min, preferably 0.1 °C/min to 10°C/min, and more preferably 0.1°C/min to 3.3°C/min, and the alloy is retained [Step 5-6] in this temperature range [14] for 20 to 480 minutes, and preferably 30 to 360 minutes [15]. Then, heating [Step 5-7] is performed again from the temperature range [14] for obtaining the ($\alpha$+$\beta$) phase (for example, 450°C) to the temperature range [17] for obtaining the $\beta$ single phase (for example, 900°C) at the speed of temperature raising [16] of the slow temperature raising, and the alloy is retained [Step 5-8] in this temperature range [17] for 5 to 480 minutes, and preferably 10 to 360 minutes [18].

Repetition [Step 5-9] of such a [Step 5-4] to [Step 5-8] (conditions [11] to [18]) is carried out at least one time and preferably at least four times [19].

The cooling speed [20] at the time of rapid cooling [Step 5-10] is usually set to 30°C/sec or more, preferably 100°C/sec or more, and more preferably 1,000°C/sec or more.

The final optional aging heat treatment [Step 6] is usually carried out at 100°C to 200°C [21] for 5 to 120 minutes [22], and preferably at 120°C to 200°C [21] for 5 to 120 minutes [22].

Furthermore, the hallux valgus correction device of the present invention also enables the correction to be achieved comfortably and steadily in a stepwise manner using a sheet having a small thickness (weak load) in the early stage, a sheet having a slightly large thickness (moderate load) in the intermediate stage, and a sheet with a large thickness (heavy load) in the final stage, by accommodating superelastic sheets (corrector, 10) in an insertable and removable storage bag (corrector storage unit) (6).

Moreover, when the hallux valgus correction device of the present invention is used, since the correction device can be worn for a long time or for a long time period, the correction device is also applicable to conservative therapy.

EXAMPLES

The present invention will be described in more detail based on examples given below, but the invention is not meant to be limited by these.

As an Example, the hallux valgus correction device of the present invention illustrated in Fig. 1 to Fig. 3 was used. Specifically, the hallux valgus correction device 1 of the present invention includes a corrector 10 as well as a hinge 11, a fastening band 2, a second annular bandage (fixture) 3, a first annular bandage (fixture) 4, a cushioning member 5, and a corrector storage bag 6.

The corrector (sheets made of a superelastic alloy) 10 of the hallux valgus correction device of the present invention was produced by the following method. A mixed material of 8.1 mass% of Al and 11.1 mass% of Mn, with the balance being Cu, was melted and cast in a high-frequency vacuum melting furnace, and the resultant material was subjected to hot-forging at 800°C and hot-rolling at 600°C, followed by repeatedly subjected to intermediate annealing at 520°C and cold-rolling at a cold-working ratio of 40%. Thus, a sheet having a sheet thickness of 0.4 mm was produced. The sheet was punched into a predetermined shape, and the resultant punched sheet was heated to 500°C at a speed of temperature raising of 10°C/min in an electric furnace, maintained at 500°C for one hour, heated to 900°C at a speed of temperature raising of 1.0°C/min, and maintained for 10 minutes at 900°C. Then, the sheet was cooled to 500°C at a speed of temperature lowering of 1.0°C/min, maintained at 500°C for one hour, then heated to 900°C at a speed of temperature raising of 1.0°C/min, maintained for one hour, and then rapidly cooled in water. Then, the resultant sheet was subjected to aging treatment at 130°C for a heat treatment time of 30 minutes, and thus, superelasticity was exhibited. In this manner, a corrector (sheets made of a superelastic alloy) was obtained.

Regarding the fastening band 2, use was made of: a belt manufactured by MJ NEOPRENE CO., LTD, in which to one end of a fabric rubber having one surface covered with the loops of a hook-and-loop fastener, a metallic fitting for passing the hooks of the hook-and-loop fastener is attached, and the loops of the hook-and-loop fastener are attached to the other end.

Regarding the second annular bandage 3, use was made of: a tape-like fabric rubber having a width of 50 mm to 100 mm and having one surface covered with a fabric of an extra-fine denier polyester fiber (manufactured by TEIJIN LIMITED, NANOFRONT (trade name)), and the metatarsal circumference diameter of each of the monitors was measured. The fabric rubber was sewed together such that the length was shorter by about 5% than the circumference diameter in the direction in which the fabric rubber would stretch in the metatarsal circumferential direction. At the time of sewing together, the loops of the hook-and-loop fastener for attaching the annular bandage and the corrector storage bag were kept in a sewed state. The tape width was adjusted as appropriate in accordance with the size of the monitor's metatarsus.

Regarding the first annular bandage 4, use was made of: a tape-like fabric rubber having a width of 20 mm to 40 mm and having one surface covered with a fabric of an extra-fine denier polyester fiber (manufactured by TEIJIN LIMITED, NANOFRONT). The hooks of the hook-and-loop fastener were attached to one end of the fabric rubber, and the loops of the hook-and-loop fastener were attached to the other end of the fabric rubber, so that the bandage could be applied to the big toes of individual monitors having different big toe sizes.

Regarding the cushioning member (big-toe-base inner-side protrusion cushioning pad) 5, a member made of neoprene was used.

The corrector storage bag 6 was made of neoprene, and a fabric rubber having one surface covered with the hooks of a hook-and-loop fastener was sewed into a bag shape such that the hook-and-loop fastener would be exposed at the surface. The surfaces of protruding inner sides of the opening of the bag were sewed with the hooks and the loops of the hook-and-loop fastener so that the corrector would not come out.

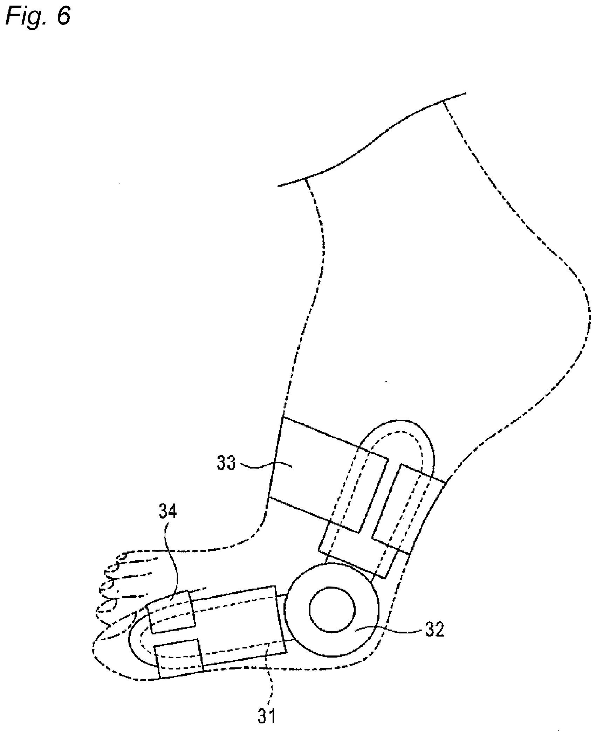

Regarding Comparative Examples, used were, respectively, a conventional hallux valgus correction device illustrated in Fig. 5, and a conventional hallux valgus correction device illustrated in Fig. 6 (manufactured by Hallufix AG).

These hallux valgus correction devices of Examples and Comparative Examples were, respectively, used and applied to fourteen (14) hallux valgus patient monitors, and thus the correction devices were evaluated for the following items.

- Pain and comfort.
- Wearing time in sleep and in the daytime, and wearing period
- Wearing stability in the nighttime and in the daytime
- Time period required for smoothening of the MTP joint
- Degree of improvement in the angle of hallux valgus
- State of arch formation (degree of improvement) checked by a foot printer

For each of the items, the correction devices were rated as excellent "⊙", satisfactory "○", acceptable "Δ", or unacceptable or poor "×". A correction device rated as Δ or × for even a single item was considered inappropriate (poor) as the hallux valgus correction device.

The results for the above evaluation are presented in the following table.

Table 1

| | Monitors | Continuity | | | | Wearing stability | | Improvement | | |
|---|---|---|---|---|---|---|---|---|---|---|
| | | Pain | Comfort | Wearing time | Wearing period | In the nighttime | In the daytime | Smoothening of MTP joint | Angle of hallux valgus | Arch formation |
| C Ex 1 | Man right | ○ | △ | ○ | △ | △ | ○ | △ | △ | △ |
| C Ex 2 | Woman left | ○ | × | ○ | × | △ | × | ○ | △ | △ |
| Ex | Man left | ○ | ◉ | ◉ | ◉ | ○ | ○ | ◉ | ◉ | ◉ |
| | Woman right | ◉ | ◉ | ◉ | ○ | ○ | ○ | ◉ | ◉ | ◉ |
| | Woman left | ◉ | ◉ | ○ | ◉ | ○ | ○ | ◉ | ◉ | ◉ |
| | Woman right | ○ | ◉ | ◉ | ◉ | ◉ | ○ | ○ | ○ | ○ |
| | Woman left | ◉ | ◉ | ◉ | ◉ | ○ | ○ | ◉ | ◉ | ◉ |
| | Woman left | ◉ | ◉ | ◉ | ◉ | ◉ | ○ | ○ | ○ | ○ |
| | Woman right | ◉ | ◉ | ◉ | ◉ | ◉ | ○ | ◉ | ◉ | ◉ |
| | Woman left | ○ | ◉ | ◉ | ◉ | ○ | ○ | ○ | ○ | ○ |
| | Woman left | ◉ | ◉ | ◉ | ○ | ○ | ○ | ○ | ○ | ○ |
| | Woman right | ○ | ◉ | ○ | ○ | ○ | ○ | ○ | ○ | ○ |
| | Woman right | ○ | ◉ | ○ | ○ | ○ | ○ | ◉ | ◉ | ◉ |
| | Woman left | ○ | ◉ | ◉ | ○ | ○ | ○ | ◉ | ◉ | ◉ |

Note: 'C Ex 1' means Comparative Example 1,
'C Ex 2' means Comparative Example 2, and
'Ex' means Example(s) according to this invention.

| Evaluation | | ◉ | ○ | △ | × |
|---|---|---|---|---|---|
| | Pain | Does not feel any pain | Does feel a little pain | Felt a pain but stand with it | Cannot stand with a pain |
| Continuity | Comfort | Does not feel any sweaty | Does feel a little sweaty | Be sweaty | Does not wear till the morning |
| | Wearing time | 6 hours or longer | 4 hours or longer | 2 hours or longer | less than 2 hours |
| | Wearing period | 3 months or longer | 1 month or longer | Retired within 1 month | Retired within 1 week |
| Wearing stability | Nighttime | Be not dislocated at all | Dislocated a little in sleeping | Wearing again a few times in sleeping | Be off when waken up |
| | Daytime | Able to walk with the shoes | Able to walk indoors | Difficulty in walking | Dis-able to walk |
| Improvement | Angle of hallux valgus | Improved at 5° or more | Improved at 2° or more but less than 5° | Improved at less than 2° | Worsened |
| | Arch formation | Arch was formed sufficiently | Arch was improved | No change | Worsened |
| | Smoothening of joint | Contracture was improved within 1 week | Contracture was improved within 1 month | No change | Worsened |

Comparative Example 1   Fig. 6 (Japanese Patent No. 4917752)
Comparative Example 2   Fig. 5 (Japanese Patent No. 5369321)
Example(s)   Figs. 1 to 3

From the results shown in Table 1, the following is clearly understood.

When the hallux valgus correction devices of Comparative Examples 1 and 2 were used, the comprehensive evaluation results were poor. Particularly, the wearability in the nighttime was poor, and specifically, the correction devices got sweaty or easily dislocated. That is, due to a slight feeling of strangeness (discomfort) at the time of wearing, it was difficult to wear the correction devices in the nighttime (in sleep) for a long time (for example, 8 hours or longer).

On the other hand, in the Examples according to the present invention, excellent results were obtained for all of the evaluation items, and seven people out of fourteen monitors saw an improvement of 5° or more in the angle of hallux valgus (an improvement was recognized in Fig. 4(B) after use, compared to Fig. 4(A) obtained before use of the hallux valgus correction device of the present invention).

Having described our invention as related to the present embodiments, it is our intention that the invention not be limited by any of the details of the description, unless otherwise specified, but rather be construed broadly within its spirit and scope as set out in the accompanying claims.

This application claims priority on Patent Application No. 2016-132096 filed in Japan on July 1, 2016, which is entirely herein incorporated by reference.

REFERENCE SIGNS LIST

> 1 hallux valgus correction device
> 2 fastening band (metatarsal bone fastening belt)
> 3 fixture (second annular bandage)
> 4 fixture (first annular bandage)
> 5 cushioning member (big-toe-base inner-side protrusion cushioning pad)
> 6 corrector storage bag
> 10 corrector (sheet made of superelastic alloy)
> 11 hinge part
> R angle of hallux valgus
> 21 splint (correction sheet, correction sheet made of superelastic alloy)
> 22 space
> 23 annular fastening device (for metatarsal bone)
> 24 annular fastening device (for big toe)
> 25 hook-and-loop fastener
> 31 splint with spring property (typically, made of plastics)
> 32 hinge
> 33 second annular fastening device
> 34 first annular fastening device

**Claims**

1.  A hallux valgus correction device for correcting hallux valgus,
    the hallux valgus correction device including:

    > a corrector made of a superelastic alloy; and
    > a fixture formed from a fabric to attach the corrector,
    > in which the corrector has a hinge part that is rotationally movable in the bending direction and the stretching direction of one toe or a plurality of toes in need of correction.

2.  The hallux valgus correction device according to claim 1, in which the corrector made of the superelastic alloy is formed from a Cu-Al-Mn-based superelastic alloy.

3.  The hallux valgus correction device according to claim 1 or 2, in which the fixture has a first annular bandage enclosing the big toe; a second annular bandage enclosing the metatarsus; and a corrector storage unit that extends over the first annular bandage and the second annular bandage and accommodates the corrector in the inside.

4.  The hallux valgus correction device according to any one of claims 1 to 3, wherein in the fabric, a portion that comes into direct contact with the skin is formed from an extra-fine denier resin fiber.

5. The hallux valgus correction device according to any one of claims 1 to 4, in which the fixture includes a cushioning member between the hinge part and the big toe joint.

6. The hallux valgus correction device according to any one of claims 1 to 5, in which the corrector is comprised of a Cu-Al-Mn-based superelastic alloy having a composition containing 3.0 to 10.0 mass% of Al, and 5.0 to 20.0 mass% of Mn, with the balance being Cu and unavoidable impurities, and capable of containing, as an optional additional element, 0.000 to 10.000 mass% in total of at least one selected from the group consisting of Ni, Co, Fe, Ti, V, Cr, Si, Nb, Mo, W, Sn, Mg, P, Be, Sb, Cd, As, Zr, Zn, B, C, Ag, and misch metal (Pr, Nd, and the like).

*Fig. 1*

*Fig. 2*

*Fig. 3*

## Fig. 4(A)
Before wearing

## Fig. 4(B)
After wearing for 60 days

## Fig. 5

Fig. 6

## Fig. 7

x means: Shape restoring force = Correction force

## Fig. 8

| Step 1 | [1] | Melting and casting | Step 5-1 | [7] | RT ⇒ (α+β) temp. raising speed | Step 5-6 | [14] | (α+β) temp. |
|---|---|---|---|---|---|---|---|---|
| Step 2 | [2] | Hot-working temp. | Step 5-2 | [8] | (α+β) temp. | | [15] | (α+β) time |
| Step 3 | [3] | Intermediate annealing temp. | | [9] | (α+β) time | Step 5-7 | [16] | (α+β) ⇒β temp. raising speed |
| | [4] | Intermediate annealing time | Step 5-3 | [10] | (α+β) ⇒β temp. raising speed | Step 5-8 | [17] | β phase temp. |
| Step 4-1 | [5] | Working ratio in cold-working | Step 5-4 | [11] | β phase temp. | | [18] | β phase time |
| Step 4-2 | [6] | The repetition number in [3] to [5] (cumilative working ratio) | | [12] | β phase time | Step 5-9 | [19] | The repetition number in [11] to [18] |
| | | | Step 5-5 | [13] | β ⇒(α+β) temp. lowering speed | Step 5-10 | [20] | Cooling speed |
| | | | | | | Step 6 | [21] | Aging temp. |
| | | | | | | | [22] | Aging time |

Note: RT is room temperature

## INTERNATIONAL SEARCH REPORT

| International application No. |
| --- |
| PCT/JP2017/024092 |

A. CLASSIFICATION OF SUBJECT MATTER
*A61F5/10*(2006.01)i, *A61F5/01*(2006.01)i, *A61F5/02*(2006.01)i

According to International Patent Classification (IPC) or to both national classification and IPC

B. FIELDS SEARCHED

Minimum documentation searched (classification system followed by classification symbols)
A61F5/10, A61F5/01, A61F5/02

Documentation searched other than minimum documentation to the extent that such documents are included in the fields searched
Jitsuyo Shinan Koho          1922–1996     Jitsuyo Shinan Toroku Koho     1996–2017
Kokai Jitsuyo Shinan Koho     1971–2017     Toroku Jitsuyo Shinan Koho     1994–2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 4917752 B2  (Huber Vitus Maria),<br>18 April 2012 (18.04.2012),<br>paragraphs [0010] to [0071]; fig. 1 to 21<br>& US 2006/0155233 A1<br>paragraphs [0032] to [0092]; fig. 1 to 21<br>& WO 2004/019835 A2      & EP 1531768 A2<br>& DE 203012159 U1      & AT 324090 T<br>& HK 1080280 A        & ES 2263055 T<br>& CN 1697635 A        & AU 2003251638 A<br>& ES 2486866 T | 1–6 |

| ☒ | Further documents are listed in the continuation of Box C. | ☐ | See patent family annex. |
| --- | --- | --- | --- |

| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 September 2017 (22.09.17) | Date of mailing of the international search report<br>03 October 2017 (03.10.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
|---|
| PCT/JP2017/024092 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 5369321 B2  (Furukawa Techno Material Co., Ltd.), 18 December 2013 (18.12.2013), paragraphs [0011] to [0022]; fig. 1 to 13 & US 2013/0110024 A1    & WO 2011/086716 A1 & EP 2524675 A1 paragraphs [0011] to [0022]; fig. 1 to 13 | 1-6 |
| Y | JP 2014-198159 A  (Descente, Ltd.), 23 October 2014 (23.10.2014), paragraphs [0010] to [0050]; fig. 1 to 6 (Family: none) | 4-6 |
| A | JP 10-290816 A  (Ota Kasei Kogyo Yugen Kaisha), 04 November 1998 (04.11.1998), (Family: none) | 1-6 |
| A | JP 3191779 U  (Alcare Co., Ltd.), 10 July 2014 (10.07.2014), (Family: none) | 1-6 |

Form PCT/ISA/210 (continuation of second sheet) (January 2015)

**EP 3 479 801 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4917752 B
- JP 5369321 B

- JP 2016132096 A